(19) Europäisches Patentamt

European Patent Office

Office européen des brevets

(11) **EP 1 693 444 A1**

(12) **EUROPEAN PATENT APPLICATION**

(43) Date of publication:
**23.08.2006 Bulletin 2006/34**

(51) Int Cl.:
*C12M 3/00* (2006.01)    *C12N 15/89* (2006.01)

(21) Application number: **06250278.6**

(22) Date of filing: **19.01.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **17.02.2005 JP 2005041032**
**24.08.2005 JP 2005243371**

(71) Applicant: **FUJITSU LIMITED**
**Kawasaki-shi,**
**Kanagawa 211-8588 (JP)**

(72) Inventors:
• **Youoku, Sachihiro,**
**Fujitsu Limited**
**Kawasaki-shi**
**Kanagawa 211-8588 (JP)**

• **Ando, Moritoshi,**
**Fujitsu Limited**
**Kawasaki-shi**
**Kanagawa 211-8588 (JP)**
• **Ito, Akio,**
**Fujitsu Limited**
**Kawasaki-shi**
**Kanagawa 211-8588 (JP)**

(74) Representative: **Hitching, Peter Matthew et al**
**HASELTINE LAKE**
**Imperial House**
**15-19 Kingsway**
**London WC2B 6UD (GB)**

(54) **Substance injecting apparatus**

(57)     A particle fixing unit fixes a particle (19). A needle (12) is arranged in such a manner that an angle between a surface on a side of the particle fixing unit on which the particle is fixed and a longitudinal center axis of the needle becomes equal to or more than 45 degrees and equal to or less than 135 degrees. An image forming unit forms, when observing at least one of the particle and the needle across the particle fixing unit, at least one of an image of the particle and an image of the needle (12), based on a light transmitted through the particle fixing unit.

FIG.1

EP 1 693 444 A1

**Description**

BACKGROUND OF THE INVENTION

1. Field of the Invention

The present invention relates to a technology for preventing an interference between a needle for injecting a substance into a cell and an observation lens, increasing a success rate in inserting the needle into the cell, and facilitating an observation of the needle and the cell, without increasing a production cost of an apparatus.

2. Description of the Related Art

Recently, in the field of life science, particularly in the fields of regenerative medicine and genome-based drug discovery, it is common to inject a gene or a drug into a cell to alter the property of the cell. The use of such technology enables elucidation of the role of genes, and customized medicine for conducting treatment according to individual genetics.

When a gene or a drug is injected into a cell, it is necessary to fix the cell not to move. Therefore, a technique has been developed, in which a cell-fixing plate with a hole smaller than the diameter of the cell is used, and the cell is attracted thereto by a suction pump, to fix the cell at the hole (see, for example, Japanese Patent No. 2624719 and Japanese Patent Application Laid-open No. 2003-419091).

Furthermore, a microinjection method using an electrophoresis has been developed as a method of injecting a gene or a drug into a cell. In this microinjection method, since a needle is used to precisely inject a trace of a sample in the needle into the cell, the amount of shift of the sample in the needle is controlled by using the electrophoresis (see, for example, Japanese Patent Application Laid-open No. H5-192171).

In the conventional techniques, however, the cell moves when the needle is inserted into the cell for injecting a gene or a drug while observing the cell. Specifically, the needle needs to be pressed against the cell to open a hole in the cell. However, if an angle between a surface of the cell-fixing plate on which the cell is fixed and a center axis of the needle is small, the cell moves, making it difficult to insert the needle.

Furthermore, in the conventional techniques, when the cell and the needle for injecting a gene or a drug into the cell are to be diascopically observed by using an observation device having an inverted type optical system, a device that can easily perform a diascopic observation cannot be produced at a low cost.

When a cell or a needle is diascopically observed with the observation device having the inverted type optical system, it is necessary to form the portion for fixing the cell with a transparent material, and hence, the manufacturing and machining method thereof is limited.

For example, polystyrene is generally used as the transparent material, and it is necessary to perform laser beam machining in order to bore a hole for fixing a cell through polystyrene. Since it takes time and labor, mass production becomes difficult, thereby increasing the production cost of the device.

If cells are observed with an observation device having an erecting optical system, cells and needles can be easily observed. However, in this case, since the needle and an observation lens interfere with each other, the installation angle of the needle is limited, and hence, observations cannot be performed easily.

Accordingly, there is a demand for development of a technique that facilitates observations of cells and needles while preventing interference between the needle and an observation lens, increasing a success rate in inserting the needle into the cell, and without increasing the production cost thereof.

SUMMARY OF THE INVENTION

It is an object of the present invention to at least solve the problems in the conventional technology.

An apparatus for injecting a substance into a particle, according to one aspect of the present invention, includes a particle fixing unit that fixes the particle; a needle that is arranged in such a manner that an angle between a surface on a side of the particle fixing unit on which the particle is fixed and a longitudinal center axis of the needle becomes equal to or more than 45 degrees and equal to or less than 135 degrees, to inject the substance into the particle by forming a hole in the particle; and an image forming unit that forms, when observing at least one of the particle and the needle across the particle fixing unit, at least one of an image of the particle and an image of the needle, based on a light transmitted through the particle fixing unit.

A method of injecting a substance into a particle, according to another aspect of the present invention, includes fixing the particle on a particle fixing unit; arranging a needle in such a manner that an angle between a surface on a side of the particle fixing unit on which the particle is fixed and a longitudinal center axis of the needle becomes equal to or more than 45 degrees and equal to or less than 135 degrees; and forming, when observing at least one of the particle and the needle across the particle fixing unit, at least one of an image of the particle and an image of the needle, based on a light transmitted through the particle fixing unit.

The above and other objects, features, advantages and technical and industrial significance of this invention will be better understood by reading the following detailed description of presently preferred embodiments of the invention, when considered in connection with the accompanying drawings.

BRIEF DESCRIPTION OF THE DRAWINGS

Fig. 1 is a schematic of a substance injecting apparatus according to the present invention;

Fig. 2 is a schematic of a functional configuration of the substance injecting apparatus according to the present invention;

Fig. 3 is a schematic of a configuration example of an illumination when irradiating a long-wavelength light having a wavelength longer than that of a red light and a short-wavelength light having a wavelength shorter than that of a green light to a trapping chip formed of a material such as Si or $SiO_2$;

Fig. 4 is a schematic of an example of a ring illumination that emits the long-wavelength light and the short-wavelength light;

Fig. 5 is a schematic of an example of a configuration with a long-wavelength light transmission filter and a short-wavelength light transmission filter; and

Fig. 6 is a schematic of an example of a configuration with the long-wavelength light transmission filter and the short-wavelength light transmission filter installed on an objective lens side.

DETAILED DESCRIPTION OF THE PREFERRED EMBODIMENTS

Exemplary embodiments of the present invention are explained in detail with reference to the accompanying drawings. According to the embodiments, a case in which the present invention is applied to a substance injecting apparatus that injects a substance such as a gene or a drug into a cellular particle is explained.

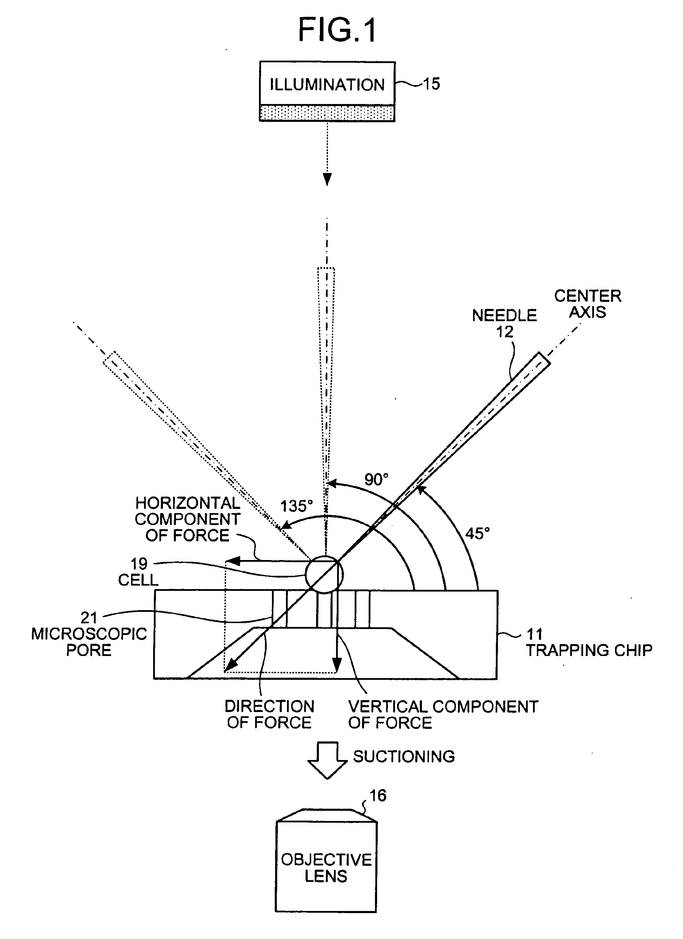

Fig. 1 is a schematic of a substance injecting apparatus according to the present invention. The substance injecting apparatus traps a cell 19 so as not to move when injecting a substance into the cell 19. Therefore, a microscopic pore 21 (opening) is formed in a trapping chip 11 on which the cell 19 is mounted, and the cell 19 is sucked from the microscopic pore 21.

In the substance injecting apparatus, a needle 12 that injects a substance in the cell 19 is adjusted such that an angle between a surface on a side of the trapping chip 11 on which the cell 19 is fixed and a center axis of the needle 12 is 45 degrees or more and 135 degrees or less when the needle 12 is inserted in the cell 19 to form a hole in the cell 19.

When the angle of the needle 12 is adjusted as above, force of a vertical component on the cell 19 becomes larger than force of a horizontal component when the needle is inserted into the cell 19. Therefore, the cell 19 is prevented from moving horizontally, so that a success rate in inserting the needle 12 into the cell 19 is increased.

An illumination 15 is provided over the trapping chip 11 where the cell 19 and the needle 12 are located, and an objective lens 16 is used to observe the cell 19 and the needle 12 through the trapping chip 11. Therefore, interference between the needle 12 and the objective lens 16 can be prevented.

When the needle 12 is inserted into the cell 19, as shown in Fig. 1, the direction of the center axis of the needle 12 is aligned with the center direction of the cell 19, and the needle 12 is pushed out towards the center of the cell 19 to be inserted into the cell 19. Alternatively, the needle 12 can be pressed against the cell 19 from above to be inserted into the cell 19. In this case, it is preferable to insert the needle 12 at a high angle as much as possible so that the hole is not too large.

In this example, the angle of the needle 12 is adjusted such that the angle between the surface on the side of the trapping chip 11 on which the cell 19 is fixed and the center axis of the needle 12 is 45 degrees or more and 135 degrees or less. However, when the angle is 60 degrees or more and 120 degrees or less, the needle 12 can be inserted into the cell 19 more reliably. When the angle is 90 degrees, it is difficult to observe the needle 12. Therefore, the angle can be adjusted to be at any angle other than 90 degrees.

According to each of the following embodiments, the angle of the needle 12 is adjusted such that the angle between the surface on the side of the trapping chip 11 on which the cell 19 is fixed and the center axis of the needle 12 is 45 degrees or more and 135 degrees or less when a hole is formed in the cell 19.

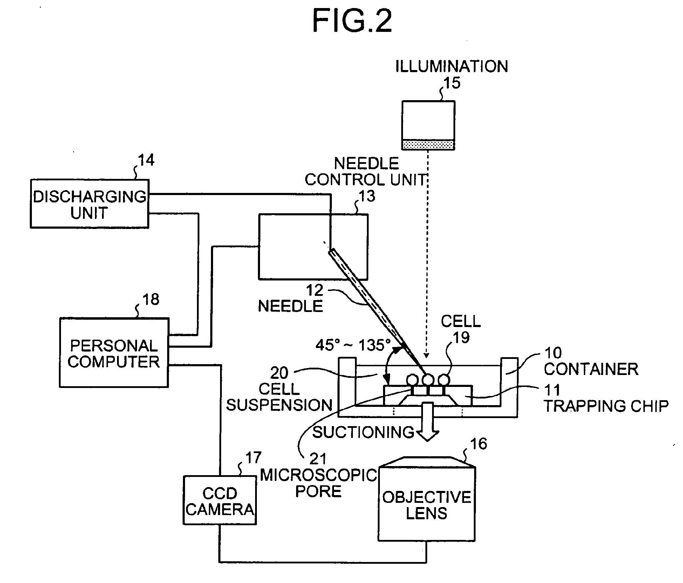

Fig. 2 is a schematic of a functional configuration of the substance injecting apparatus according to the present invention.

The trapping chip 11 shown in Fig. 2 is manufactured by using a substance, of which light transmittance changes according to the wavelength of the light, such as Si or $SiO_2$. By using light of a wavelength having a high light transmittance, even if the optical system in the substance injecting apparatus is an inverted type, the cell 19 and the needle 12 for injecting the substance into the cell 19 can be easily observed across the trapping chip 11.

When the optical system is the inverted type, the needle 12 for injecting the substance into the cell 19 can be installed at a high angle with respect to the trapping chip 11, thereby increasing the flexibility of the installation angle of the needle 12. Accordingly, the success rate in inserting the needle 12 into the cell 19 can be increased.

When Si or $SiO_2$ is used as the material of the trapping chip 11, mass production of the trapping chip 11 becomes possible by process machining at a low cost. Furthermore, fine machining becomes possible. Accordingly, the substance injecting apparatus that enables diascopic observations of the cells 19 and the needles 12 can be manufactured.

The substance injecting apparatus includes a container 10, the trapping chip 11, the needle 12, a needle control unit 13, a discharging unit 14, the illumination 15, the objective lens 16, a CCD camera 17, and a personal computer 18.

The container 10 stores a cell suspension 20 in which the cells 19 are contained in a solution. The trapping chip 11 is a chip in which the microscopic pore 21 having a diameter smaller than that of the cell 19 is formed, to attract the cell 19 by applying a negative pressure to the microscopic pore 21 by using a pump (not shown) or the like and trap the cell 19. It is desired that the diameter of the microscopic pore 21 be equal to or smaller than 5 micrometers, but it is not limited thereto.

A method of supplying the cell 19 to the trapping chip 11 can be by dropping the cell suspension 20 containing the cell 19 onto the trapping chip 11, using a slender pipe such as a pipette, or by forming a flow path for flowing the cell suspension 20 to the trapping chip 11. Alternatively, the cell 19 can be supplied to the trapping chip 11 by setting the trapping chip 11 in the container 10 and using a supply apparatus that supplies the cell suspension 20 to the container 10.

The trapping chip 11 is manufactured by using a material, of which light transmittance changes according to the wavelength of light, such as Si or $SiO_2$. Si or $SiO_2$ has a property such that the light transmittance is high with respect to a long-wavelength light having a wavelength longer than that of a red light, more specifically, with respect to the light having a wavelength equal to or longer than 650 nanometers.

Accordingly, when the light having the long-wavelength light to the trapping chip 11 formed of the substance such as Si or $SiO_2$, the cell 19, the needle 12, or the like can be diascopically observed across the trapping chip 11.

The container 10 is formed such that the light irradiated to the trapping chip 11 can penetrate the container 10. Specifically, a part of the container 10 under the trapping chip 11 can be an opening, or can be covered with a substance that transmits the light irradiated by the illumination 15.

The needle 12 is a needle for forming a minute hole in the cell 19 to inject a substance such as a gene or a drug therefrom. The needle control unit 13 is a control unit that controls the position and the like of the needle 12. The discharging unit 14 is an apparatus that supplies the substance such as a gene or a drug to be injected into the cell 19, and also controls a feed rate of the substance.

The illumination 15 is the one that emits the long-wavelength light, preferably, the light having a wavelength equal to or longer than 650 nanometers. The objective lens 16 is the one that collects the light emitted from the illumination 15 from under the trapping chip 11.

The CCD camera 17 is a camera that receives the light collected by the objective lens 16 to form an image. The personal computer 18 is a computer that controls the operation of the needle control unit 13, the discharging unit 14, and the CCD camera 15 and stores the formed image in a memory or the like, to perform analysis of the image.

Observation of the microscopic pores 21 formed in the trapping chip 11 can be performed by using an illumination that emits a short-wavelength light having a wavelength shorter than that of a green light, using the property of Si or $SiO_2$, which has a low light transmittance with respect to a light having a wavelength equal to or shorter than 550 nanometers.

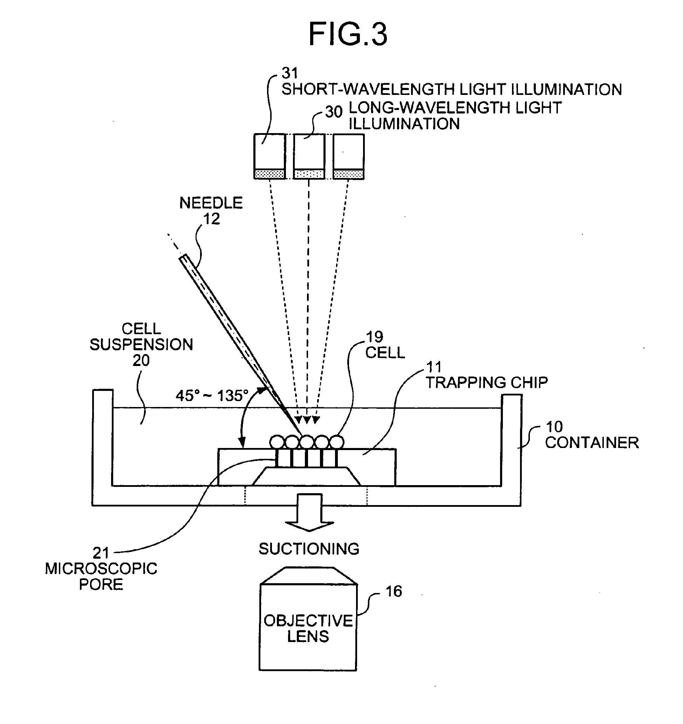

Fig. 3 is a schematic of a configuration example of an illumination when irradiating the long-wavelength light and the short-wavelength light to a trapping chip 11 formed of a material such as Si or $SiO_2$.

In this configuration example, the illumination includes a long-wavelength light illumination 30 that emits the long-wavelength light, preferably, a light having a wavelength equal to or longer than 650 nanometers, and a short-wavelength light illumination 31 that emits the short-wavelength light, preferably, a light having a wavelength equal to or shorter than 550 nanometers. The short-wavelength light illumination 31 is a ring illumination installed so as to surround the long-wavelength light illumination 30.

The container 10, the trapping chip 11, the needle 12, and the objective lens 16 shown in Fig. 3 are the same as those shown in Fig. 2, and although not shown in Fig. 3, the configuration of the needle control unit 13, the discharging unit 14, the CCD camera 17, the personal computer 18, and the like are the same as that shown in Fig. 1.

When the long-wavelength light illumination 30 irradiates the long-wavelength light onto the trapping chip 11 formed of a material such as Si or $SiO_2$, the cell 19, the needle 12, and the like can be diascopically observed across the trapping chip 11, as described above.

On the other hand, when the short-wavelength light illumination 31 irradiates the short-wavelength light onto the trapping chip 11, since the light transmittance of the trapping chip 11 is low, the microscopic pores 21 formed in the trapping chip 11 can be observed.

In this case, the personal computer 18 performs image processing with respect to the image formed by using the long-wavelength light, and the image formed by using the short-wavelength light, and performs processing for identifying the parts of the cell 19 and the needle 12, and the microscopic pores 21 formed in the trapping chip 11.

In the short-wavelength light illumination 31, since the wavelength of light to be emitted is short, the resolution can be improved, and the precision of the image processing in the depth direction such as automatic focus can be improved.

The relation between the wavelength of light and the resolution is expressed by

$$(\text{Resolution}) \fallingdotseq 0.61\lambda/\text{NA}$$

where $\lambda$ is the wavelength, and NA is a numerical aperture of the lens.

The relation between the wavelength of light and the depth of focus is expressed by

$$(\text{Depth of focus}) \fallingdotseq \pm\lambda/(2\text{NA}\cdot\text{NA})$$

Therefore, by using the light having a wavelength equal to or shorter than 550 nanometers, the resolution can be improved and the precision of the image processing in the depth direction can be improved. Since the short-wavelength light illumination 31 is a ring illumination, the incident angle of the light to the microscopic pores 21 can be increased, thereby simplifying the determination of height of the microscopic pores 21.

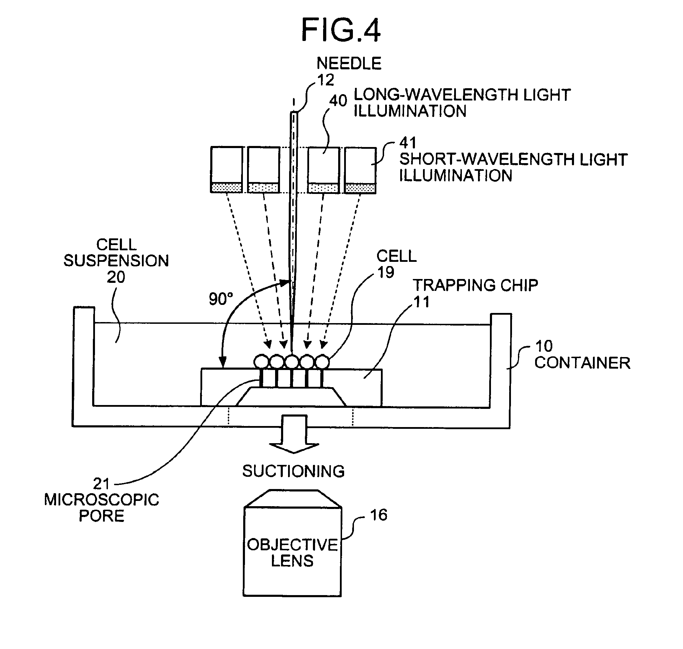

The short-wavelength light illumination 31 is the ring illumination in the example shown in Fig. 3, but the long-wavelength light illumination 30 can be also the ring illumination. Fig. 4 is a schematic of an example of a ring illumination that emits the long-wavelength light and the short-wavelength light.

In the configuration example shown in Fig. 4, the illumination includes a long-wavelength light illumination 40 being the ring illumination that emits the long-wavelength light, preferably, light having a wavelength equal to or longer than 650 nanometers, and a short-wavelength light illumination 41 being the ring illumination that emits the short-wavelength light, preferably, the light having a wavelength equal to or shorter than 550 nanometers.

The container 10, the trapping chip 11, the needle 12, and the objective lens 16 shown in Fig. 4 are the same as the container 10, the trapping chip 11, the needle 12, and the objective lens 16 shown in Fig. 2. Although not shown in Fig. 4, the configurations of the needle control unit 13, the discharging unit 14, the CCD camera 17, the personal computer 18, and the like are the same as that in Fig. 2.

As shown in Fig. 4, the needle 12 can be installed perpendicularly with respect to the trapping chip by making the long-wavelength light illumination 40 and the short-wavelength light illumination 41 the ring illumination, thereby improving the success rate in inserting the needle 12 into the cell 19.

Both of the long-wavelength light illumination 40 and the short-wavelength light illumination 41 are installed in the example shown in Fig. 4, but only the long-wavelength light illumination 40 can be installed to observe the cell 19, the needle 12, and the microscopic pores 21.

According to a first embodiment of the present invention described above, the needle 12 is arranged such that an angle between a surface on a side of the trapping chip 11 on which the cell 19 is fixed and a center axis of the needle 12 is 45 degrees or more and 135 degrees or less is used to inject a substance by forming a hole in the cell 19, and an image of any one of the cell 19 and the needle 12 or both is formed based on light having transmitted through the trapping chip 11, when any one of the cell 19 and the needle 12 or both are observed across the trapping chip 11. Accordingly, when the needle 12 is inserted into the cell 19 while observing the cell 19 or the needle 12, interference between the needle 12 and the objective lens 16 can be prevented and a success rate in inserting the needle 12 into the cell 19 can be increased.

Furthermore, according to the first embodiment, the trapping chip 11 is made of a substance having a different light transmittance according to a wavelength of light, and the objective lens 16 forms an image of any one of the cell 19 and the needle 12 or both based on light of a particular wavelength having transmitted through the trapping chip 11, when any one of the cell 19 and the needle 12 or both are observed across the trapping chip 11 on which the cell 19 is fixed using the light having the particular wavelength. Accordingly, by using the substance having a different light transmittance according to the wavelength of light and forming the image of the cell 19 and the needle 12 with the light of the particular wavelength, diascopic observations of the cell 19 and the needle 12 can be easily performed without increasing the production cost of the trapping chip 11 as compared to the case of using polystyrene.

Moreover, according to the first embodiment, when any one of the cell 19 and the needle 12 or both are observed across the trapping chip 11 having the microscopic pore 21 for fixing the cell 19 formed therein by using light having a particular wavelength, an image of any one of the cell 19, the needle 12, and the microscopic pore 21 or all is formed based on the light of a particular wavelength having transmitted through any one of the trapping chip 11 and the microscopic pore 21 or both. Accordingly, by using the substance having a different light transmittance according to the wavelength of light and forming the image of the cell 19, the needle 12, and the microscopic pore 21 with the light of the particular wavelength, diascopic observations of the cell 19, the needle 12, and the microscopic pore 21 can be easily performed without increasing the production cost of the trapping chip 11.

Furthermore, according to the first embodiment, since the substance having a different light transmittance according to the light wavelength is Si or $SiO_2$, diascopic observations of the cell 19 and the needle 12 can be easily performed without increasing the production cost of the trapping chip 11, by producing the trapping chip 11 by process machining using Si or $SiO_2$.

Moreover, according to the first embodiment, the long-wavelength light illumination 30 emits the light having a wavelength equal to or longer than 650 nanometers, and when the emitted light passes through the trapping chip 11, the objective lens 16 forms an image of any one of the cell 19 and the needle 12 or both. Accordingly, diascopic observations of the cell 19 and the needle 12 can be easily performed by using the property of Si or $SiO_2$ which has a high light transmittance with respect to the light having a wavelength equal to or longer than 650 nanometers.

Furthermore, according to the first embodiment, in addition that the long-wavelength light illumination 30 emits the light having a wavelength equal to or longer than 650 nanometers, the short-wavelength light illumination 31 emits the light having a wavelength equal to or shorter than 550 nanometers, and when the emitted light passes through the microscopic pore 21 formed in the trapping chip 11, the objective lens 16 forms an image of the microscopic pore 21, based on the light having passed through the microscopic pore 21. Accordingly, the cell 19, the needle 12, and the microscopic pore 21 observed across the trapping chip 11 can be easily identified by using the property of Si or $SiO_2$, which has a low light transmittance with respect to the light having a wavelength equal to or shorter than 550 nanometers.

Moreover, according to the first embodiment, since the objective lens 16 receives the light, which has a particular wavelength and which has transmitted through the trapping chip 11, below the trapping chip 11, to form the image of any one of the cell 19 and the needle 12 or both based on the received light. Accordingly, by using an apparatus having an inverted optical system as the substance injecting apparatus, interference between the needle and the observation lens, which occurs in the erecting type device, can be prevented.

According to the first embodiment, diascopic observation is used by using the illumination that emits the long-wavelength light and the short-wavelength light. However, an optical filter for transmitting the long-wavelength light and the short-wavelength light can be used.

According to a second embodiment of the present invention, an instance in which an optical filter for transmitting the long-wavelength light and the short-wavelength light is used to observe cells, needles, and microscopic pores formed in the trapping chip will be explained.

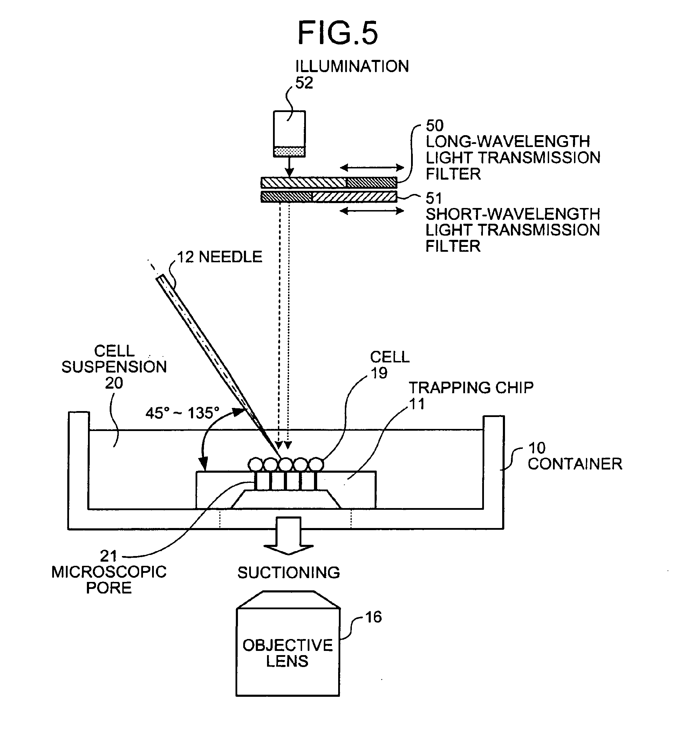

Fig. 5 is a schematic of an example of a configuration with a long-wavelength light transmission filter and a short-wavelength light transmission filter. In this configuration example, a long-wavelength light transmission filter 50, a short-wavelength light transmission filter 51, and an illumination 52 are provided.

The container 10, the trapping chip 11, the needle 12, and the objective lens 16 shown in Fig. 5 are the same as the container 10, the trapping chip 11, the needle 12, and the objective lens 16 shown in Fig. 2. Although not shown in Fig. 5, the configurations of the needle control unit 13, the discharging unit 14, the CCD camera 17, the personal computer 18, and the like are the same as that in Fig. 2.

The long-wavelength light transmission filter 50 is an optical filter that transmits the long-wavelength light, and preferably, the light having a wavelength equal to or longer than 650 nanometers. The short-wavelength light transmission filter 51 is the one that transmits the short-wavelength light, and preferably, the light having a wavelength equal to or shorter than 550 nanometers.

The illumination 52 is the one that emits light of a wide range of wavelength including a wavelength longer than that of the red light and a wavelength shorter than that of the green light. Preferably, the illumination 52 emits light in a wavelength range including wavelengths equal to or longer than 650 nanometers and wavelengths equal to or shorter than 550 nanometers.

The long-wavelength light or the short-wavelength light can be irradiated onto the cell 19, the needle 12, and the trapping chip 11, by switching the long-wavelength light transmission filter 50 and the short-wavelength light transmission filter 51. Accordingly, the cell 19, the needle 12, and the trapping chip 11 can be identified as according to the first embodiment.

In the example shown in Fig. 5, the long-wavelength light transmission filter 50 and the short-wavelength light transmission filter 51 are installed not on the objective lens 16 side but on the illumination 52 side, as seen from the trapping chip 11. However, the long-wavelength light transmission filter 50 and the short-wavelength light transmission filter 51 can be installed on the objective lens 16 side.

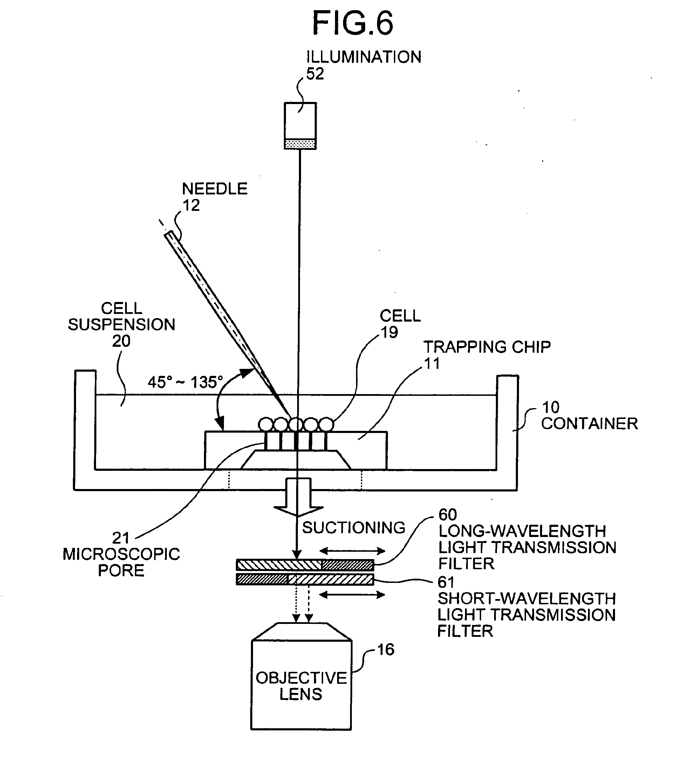

Fig. 6 is a schematic of an example of a configuration with a long-wavelength light transmission filter 60 and a short-wavelength light transmission filter 61 installed on a side of the objective lens 16. The long-wavelength light transmission filter 60 is an optical filter that transmits the long-wavelength light, and specifically, the light having a wavelength equal to or longer than 650 nanometers. The short-wavelength light transmission filter 61 is the one that transmits the short-wavelength light, and specifically, the light having a wavelength equal to or shorter than 550 nanometers.

The container 10, the trapping chip 11, the needle 12, and the objective lens 16 shown in Fig. 6 are the same as the container 10, the trapping chip 11, the needle 12, and the objective lens 16 shown in Fig. 2, the illumination 52 shown in Fig. 6 is the same as the illumination 52 shown in Fig. 5. Although not shown in Fig. 6, the configurations of the needle control unit 13, the discharging unit 14, the CCD camera 17, the personal computer 18, and the like are the same as that in Fig. 2.

Since the long-wavelength light transmission filter 60 or the short-wavelength light transmission filter 61 are used to pick out the long-wavelength light or the short-wavelength light, from lights having passed through the trapping chip 11 and the microscopic pore 21, and the objective lens 16 receives the light, the cell 19, the needle 12, and the microscopic pore 21 can be identified as according to the first embodiment.

According to the second embodiment, the objective lens 16 forms an image of any one of the cell 19 and the needle 12 or both based on the light having a particular wavelength and having passed through the trapping chip 11, the propagation of which is controlled by the long-wavelength light transmission filter 50 or 60 and the short-wavelength light transmission filter 51 or 61, which control propagation of light having a particular wavelength. Accordingly, diascopic observations of the cell 19 and the needle 12 can be easily performed by picking out the light having the particular wavelength by the long-wavelength light transmission filter 50 or 60 and the short-wavelength light transmission filter 51 or 61 and using the light.

Furthermore, according to the second embodiment, the microscopic pores 21 that fixes the particles are formed in the trapping chip 11, the trapping chip 11 is formed of Si or $SiO_2$, which is a substance having a different light transmittance according to the wavelength of light, and the long-wavelength light transmission filters 50 and 60 and the short-wavelength light transmission filters 51 and 61 respectively control so as to allow the light having a wavelength equal to or longer than 650 nanometers and the light having a wavelength equal to or shorter than 550 nanometers to propagate. Accordingly, the cell 19, the needle 12, and the microscopic pore 21 can be easily identified by using the property of Si or $SiO_2$, which has a high light transmittance with respect to the light having a wavelength equal to or longer than 650 nanometers, and a low light transmittance with respect to the light having a wavelength equal to or shorter than 550 nanometers.

While the exemplary embodiments of the present invention have been explained in detail, variously modified embodiments other than the specified ones can be also embodied in the invention, without departing from the technical spirit of the invention as defined by the appended claims.

Of the respective processing explained in the embodiments, all or a part of the processing explained as being performed automatically can be performed manually, or all or a part of the processing explained as being performed manually can be performed automatically in a known method.

The information including the processing procedure, the control procedure, specific names, and various kinds of data and parameters shown in the specification or in the drawings can be optionally changed, unless otherwise specified.

The respective constituents of the substance injecting apparatus shown in the drawings are functionally conceptual, and the physically same configuration is not always necessary. In other words, the specific mode of dispersion and integration of the substance injecting apparatus is not limited to the illustrated one and all or a part thereof can be functionally or physically dispersed or integrated in an optional unit, according to the various kinds of load and the status of use.

According to the present invention, when the needle is inserted into a particle while observing the particle or the needle, interference between the needle and an observation lens can be prevented and a success rate in inserting the needle into the cell can be increased.

Furthermore, according to the present invention, by using the substance having a different light transmittance according to the wavelength of light and forming the image of the particle and the needle with the light of the particular wavelength, diascopic observations of the particle and the needle can be easily performed without increasing the production cost of the particle fixing unit.

Moreover, according to the present invention, by using the substance having a different light transmittance according to the wavelength of light and forming the image of the particle, the needle, and the opening with the light of the particular wavelength, diascopic observations of the particle, the needle, and the opening can be easily performed without increasing the production cost of the particle fixing unit.

Furthermore, according to the present invention, since the substance having a different light transmittance according to the wavelength of the light is Si or $SiO_2$, by producing the particle fixing unit by using Si or $SiO_2$, diascopic

observations of the particle and the needle can be easily performed without increasing the production cost of the particle fixing unit.

Moreover, according to the present invention, observation of the particle and the needle can be easily performed by using the property of Si or $SiO_2$, which has a high light transmittance with respect to the light having a wavelength equal to or longer than 650 nanometers.

Furthermore, according to the present invention, the particle, the needle, and the opening can be easily identified by using the property of Si or $SiO_2$, which has a low light transmittance with respect to the light having a wavelength equal to or shorter than 550 nanometers.

Moreover, according to the present invention, observation of the particle and the needle can be easily performed by picking out and using the light having a particular wavelength, using the light propagation control unit.

Furthermore, according to the present invention, the particle, the needle, and the opening can be easily identified by using the property of Si or $SiO_2$, which has a high light transmittance with respect to the light having a wavelength equal to or longer than 650 nanometers and a low light transmittance with respect to the light having a wavelength equal to or shorter than 550 nanometers.

Moreover, according to the present invention, by constructing a device having an inverted optical system, interference between the needle and the observation lens, which occurs in the erecting type device, can be prevented.

Furthermore, according to the present invention, by using the substance having a different light transmittance according to the wavelength of light, diascopic observations of the particle and the needle can be easily performed without increasing the production cost of the particle fixing unit.

Although the invention has been described with respect to a specific embodiment for a complete and clear disclosure, the appended claims are not to be thus limited but are to be construed as embodying all modifications and alternative constructions that may occur to one skilled in the art that fairly fall within the basic teaching herein set forth.

## Claims

1. An apparatus for injecting a substance into a particle, the apparatus comprising:

   a particle fixing unit that fixes the particle;
   a needle that is arranged in such a manner that an angle between a surface on a side of the particle fixing unit on which the particle is fixed and a longitudinal center axis of the needle becomes equal to or more than 45 degrees and equal to or less than 135 degrees, to inject the substance into the particle by forming a hole in the particle; and
   an image forming unit that forms, when observing at least one of the particle and the needle across the particle fixing unit, at least one of an image of the particle and an image of the needle, based on a light transmitted through the particle fixing unit.

2. The apparatus according to claim 1, wherein
   the particle fixing unit is formed of a material having a different light transmittance depending on a wavelength of light, and
   the image forming unit forms, when observing the at least one of the particle and the needle across the particle fixing unit using a light of a specific wavelength, the at least one of the image of the particle and the image of the needle, based on the light of the specific wavelength transmitted through the particle fixing unit.

3. The apparatus according to claim 2, wherein
   the particle fixing unit includes an opening at which the particle is fixed, and
   the image forming unit forms, when observing the at least one of the particle and the needle across the particle fixing unit using a light of a specific wavelength, the at least one of the image of the particle and the image of the needle, based on the light of the specific wavelength transmitted through the opening.

4. The apparatus according to claim 2, wherein
   the material is either one of a silicon and a silicon dioxide.

5. The apparatus according to claim 4, further comprising:

   a light emitting unit that emits a long-wavelength light having a wavelength equal to or longer than 650 nanometers, wherein
   the image forming unit forms, when observing the at least one of the particle and the needle across the particle fixing unit using a light of a specific wavelength, the at least one of the image of the particle and the image of the needle, based on the light emitted from the light emitting unit and transmitted through the particle fixing unit.

6.  The apparatus according to claim 5, wherein
    the particle fixing unit includes an opening at which the particle is fixed,
    the light emitting unit further emits a short-wavelength light having a wavelength equal to or shorter than 550 nanometers, and
    the image forming unit forms, when observing the at least one of the particle and the needle across the particle fixing unit using a light of a specific wavelength, the at least one of the image of the particle and the image of the needle, based on the light emitted from the light emitting unit and transmitted through the opening.

7.  The apparatus according to claim 2, further comprising:

    an optical filter that transmits a light of a specific wavelength, wherein
    the image forming unit forms, when observing the at least one of the particle and the needle across the particle fixing unit using a light of a specific wavelength, the at least one of the image of the particle and the image of the needle, based on the light of the specific wavelength transmitted through the optical filter and the particle fixing unit.

8.  The apparatus according to claim 7, wherein
    the particle fixing unit includes an opening at which the particle is fixed,
    the material is either one of a silicon and a silicon dioxide, and
    the optical filter transmits at least one of a long-wavelength light having a wavelength equal to or longer than 650 nanometers and a short-wavelength light having a wavelength equal to or shorter than 550 nanometers.

9.  The apparatus according to claim 2, wherein
    the image forming unit receives, when observing the at least one of the particle and the needle across the particle fixing unit using a light of a specific wavelength, the light of the specific wavelength transmitted through the particle fixing unit at a position below the particle fixing unit, and forms the at least one of the image of the particle and the image of the needle based on the received light.

10. A method of injecting a substance into a particle, the method comprising:

    fixing the particle on a particle fixing unit;
    arranging a needle in such a manner that an angle between a surface on a side of the particle fixing unit on which the particle is fixed and a longitudinal center axis of the needle becomes equal to or more than 45 degrees and equal to or less than 135 degrees; and
    forming, when observing at least one of the particle and the needle across the particle fixing unit, at least one of an image of the particle and an image of the needle, based on a light transmitted through the particle fixing unit.

# FIG.1

ILLUMINATION ~15

CENTER AXIS

NEEDLE 12

90°

135°

HORIZONTAL COMPONENT OF FORCE

45°

19 CELL

21 MICROSCOPIC PORE

11 TRAPPING CHIP

DIRECTION OF FORCE

VERTICAL COMPONENT OF FORCE

SUCTIONING

16

OBJECTIVE LENS

# FIG.2

ILLUMINATION
15

DISCHARGING UNIT — 14

NEEDLE CONTROL UNIT — 13

NEEDLE — 12

PERSONAL COMPUTER — 18

CELL — 19

45° ~ 135°

CELL SUSPENSION — 20

CONTAINER — 10

TRAPPING CHIP — 11

SUCTIONING

CCD CAMERA — 17

MICROSCOPIC PORE — 21

OBJECTIVE LENS — 16

# FIG.3

31
SHORT-WAVELENGTH LIGHT ILLUMINATION

30 LONG-WAVELENGTH LIGHT
ILLUMINATION

NEEDLE
12

CELL
SUSPENSION
20

45° ~ 135°

19
CELL

11
TRAPPING CHIP

10
CONTAINER

21
MICROSCOPIC
PORE

SUCTIONING

16

OBJECTIVE
LENS

12

# FIG.4

NEEDLE
12
40 LONG-WAVELENGTH LIGHT ILLUMINATION
41 SHORT-WAVELENGTH LIGHT ILLUMINATION

CELL SUSPENSION 20

CELL 19

TRAPPING CHIP 11

90°

10 CONTAINER

SUCTIONING

21 MICROSCOPIC PORE

16

OBJECTIVE LENS

# FIG.5

ILLUMINATION
52

50
LONG-WAVELENGTH
LIGHT TRANSMISSION
FILTER

51
SHORT-WAVELENGTH
LIGHT TRANSMISSION
FILTER

12 NEEDLE

CELL
SUSPENSION
20

CELL
19

TRAPPING CHIP
11

45° ~ 135°

10
CONTAINER

21
MICROSCOPIC
PORE

SUCTIONING

16

OBJECTIVE
LENS

# FIG.6

ILLUMINATION
52

NEEDLE
12

CELL
SUSPENSION
20

CELL
19

TRAPPING CHIP
11

45° ~ 135°

10
CONTAINER

21
MICROSCOPIC
PORE

SUCTIONING

60
LONG-WAVELENGTH
LIGHT TRANSMISSION
FILTER

61
SHORT-WAVELENGTH
LIGHT TRANSMISSION
FILTER

16
OBJECTIVE
LENS

European Patent
Office

**EUROPEAN SEARCH REPORT**

Application Number

EP 06 25 0278

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 479 759 A (FUJITSU LIMITED) 24 November 2004 (2004-11-24) * paragraphs [0001], [0002], [0014], [0017], [0022], [0032] - [0034], [0037], [0072], [0073], [0078] * * claims; figures * ----- | 1,10 | INV. C12M3/00 C12N15/89 |
| A | US 2003/021017 A1 (EIJSACKERS MARCEL JOHAN ET AL) 30 January 2003 (2003-01-30) * paragraphs [0002], [0007], [0008], [0012], [0017], [0018], [0027], [0030], [0046], [0047], [0070] * * figures * ----- | 1-10 | |
| A | US 4 895 805 A (SATO ET AL) 23 January 1990 (1990-01-23) * column 1, lines 39-68 * * column 2, lines 25-35,61-68 * * claims; figures * ----- | 1,10 | |
| A | US 5 114 854 A (BERTHOLDT ET AL) 19 May 1992 (1992-05-19) * column 1, lines 8-19 * * column 2, lines 5-9 * * column 3 * ----- | 1,10 | TECHNICAL FIELDS SEARCHED (IPC) C12N C12M A01K G02B B01L |
| A | US 2002/116732 A1 (CHRISTMANN LEANDRO) 22 August 2002 (2002-08-22) * paragraphs [0017], [0018], [0021], [0063] * * claims; figures * ----- | 1,10 | |
| A,P | EP 1 591 519 A (FUJITSU LIMITED) 2 November 2005 (2005-11-02) * paragraphs [0011], [0018], [0026] * * claims; figures * ----- | 1,10 | |

The present search report has been drawn up for all claims

| Place of search | Date of completion of the search | Examiner |
|---|---|---|
| Berlin | 16 June 2006 | Böhm, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..............................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 25 0278

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2006

| Patent document cited in search report | | Publication date | Patent family member(s) | | Publication date |
|---|---|---|---|---|---|
| EP 1479759 | A | 24-11-2004 | CN | 1572877 A | 02-02-2005 |
| | | | JP | 2004344036 A | 09-12-2004 |
| | | | US | 2004235143 A1 | 25-11-2004 |
| US 2003021017 | A1 | 30-01-2003 | DE | 10136481 A1 | 20-02-2003 |
| | | | EP | 1279986 A1 | 29-01-2003 |
| | | | JP | 2003084209 A | 19-03-2003 |
| US 4895805 | A | 23-01-1990 | DE | 3829028 A1 | 09-03-1989 |
| | | | JP | 1060365 A | 07-03-1989 |
| | | | JP | 2559760 B2 | 04-12-1996 |
| US 5114854 | A | 19-05-1992 | DE | 3808531 C1 | 13-07-1989 |
| | | | EP | 0332864 A2 | 20-09-1989 |
| | | | ES | 2037889 T3 | 01-07-1993 |
| | | | JP | 1920231 C | 07-04-1995 |
| | | | JP | 2119770 A | 07-05-1990 |
| | | | JP | 6046943 B | 22-06-1994 |
| US 2002116732 | A1 | 22-08-2002 | CA | 2437238 A1 | 22-08-2002 |
| | | | EP | 1365643 A2 | 03-12-2003 |
| | | | WO | 02064727 A2 | 22-08-2002 |
| | | | US | 2006010510 A1 | 12-01-2006 |
| EP 1591519 | A | 02-11-2005 | JP | 2005312359 A | 10-11-2005 |
| | | | US | 2005244948 A1 | 03-11-2005 |

EPO FORM P0459

For more details about this annex : see Official Journal of the European Patent Office, No. 12/82